# EUROPEAN PATENT APPLICATION

(11) **EP 2 119 394 A1**
(43) Date of publication of application: **18.11.2009**
(21) Application number: 08156206.8
(22) Date of filing: 14.05.2008
(51) Int. Cl.: A61B 5/0205, A61B 5/053, G01G 19/44, A61N 1/36, A61N 1/34, A61B 5/022, A61B 5/0402, A61B 5/00, A61B 7/02

(54) **Multifunction health apparatus**

(71) Applicant: Tso, Shun-Wun, Taiwan, Province of China (TW)
(72) Inventor: Tso, Shun-Wun, Taiwan, Province of China (TW)
(74) Representative: Viering, Jentschura & Partner

(57) **Abstract**

The present invention provides a multifunction health device, which comprises a meter and a platform. The meter has two electrodes arranged separately at its two sides or both arranged at its one side, and the meter has another electrode arranged in its bottom. The platform has four electrodes arranged in its top face, and a pressure sensor arranged in its inside. With or without some parts assisting the meter can function as an ECG detector, a TENS machine, a thermometer, or a sphygmomanometer. Connecting with the platform, the meter and the platform can function as an ECG detector, a weight scale, and a body composition estimator. Further, the meter can alternatively connect with a chassis thus functioning as a second sphygmomanometer, a stethoscope, a glucose meter, or a pulse oximeter.

## Description

### Cross Reference to Related Applications

This patent application claims the benefit under 35 U.S.C. 120 as a continuation-in-part of Utility patent application Ser. No. 11/598,128, filed Nov. 13, 2006, entitled "Multifunction Health Apparatus", now abandoned. The entire disclosure of the prior application is considered part of the disclosure of the accompanying continuation application and is hereby incorporated by reference.

### BACKGROUND OF THE INVENTION

### 1. Field of the Invention

The present invention relates to a health apparatus, and more particularly, to a multifunction health apparatus.

### 2. Description of the Prior Art

Ageing and obesity almost become the leading cause of diseases. They cause some common chronic diseases such as hypertension, diabetes, and vascular disease. These diseases always need a long-term treatment, and therefore, a large cost. To reduce the medical cost, the home care provides a convenient, low-cost, and effective method assisting the patients to monitor their condition by themselves, and thus reduces the number of registers at a hospital.

Because hypertension may not cause symptoms, the patients need to have blood pressure checked regularly. Blood pressure is measured with an instrument called sphygmomanometer.

People with hypertension may have heart or vascular disease, which also need to have regular heart-condition check. Heart-condition is diagnosed by electrocardiogram. An electrocardiogram, abbreviated as ECG or EKG, is a graphic diagram recording the electrical current in the heart in a form of continuous strip graph. The ECG results providing much useful information such as, determine whether the heart is performing normally, detects electrolyte disturbances, detects abnormalities of conduction, and provide physical condition of the heart.

Unfortunately, heart and vascular disease often go hand-in-hand with diabetes. Diabetes is defined as the body does not produce or properly use insulin, a hormone needed to convert sugar, starches, and other food into energy for daily life. If diabetics can control their glucose, they will be more likely to stay healthy. Glucose meters can help the diabetics to check their glucose at home, office, or other places. It monitors whether the glucose is in balance for reducing the risk of hypoglycemia (low blood sugar) and hyperglycemia (high blood sugar).

If people suffered from hypertension, heart disease, vascular disease, or diabetics, they probably suffered from at least two of these diseases. Therefore they probably need a sphygmomanometer, an ECG tracing machine, and a glucose meter. Because almost these devices are designed for single-function use; therefore, a large cost of instrument is inevitable.

A key factor causing people diagnosed with these diseases is overweight. The way to differentiate whether people are overweight cannot range merely by weight, but need to examine the percentage of body fat (% BF) as well. A body fat scale can examine the body fat. However, additional cost is also needed.

Unhealthful people may suffer from many pains. TENS-Transcutaneous Electrical Nerve Stimulation-is a common unit, sending electrical impulses through electrodes placed on the painful site, causing a tingling sensation that reduces pain. It helps the chronic pain or the acute pain. Unlike medicine, it has only little side effects such as causing the skin irritation. Again, this useful machine consumes more cost.

Yet some other home-care units such as weight scale, thermometer, and pulse oximeter are also popular. The later helps usefully in lung disease. If there is a home care apparatus capable of measuring, detecting, computing the multiple parameters mentioned above, the cost would be dramatically decreased. More, if people can choose the functions associated in this multifunction health apparatus as they want, it would be expected to be more popular and acceptable in market.

### SUMMARY OF THE INVENTION

An object of the present invention is to provide a device capable of measuring multiple parameters of human, for decreasing the cost. Furthermore, people can choose the functions associated in this device.

According to the object, the present invention provides a multifunction health device, which comprises a meter and a platform. The meter has two electrodes arranged separately at its two sides or arranged at the same side, and the meter has another electrode arranged in its bottom. The platform has four electrodes arranged in its top face, and a loading cell arranged in its inside. With or without some parts assisting, the single use of the meter can function as an ECG detector, a TENS machine, and a thermometer. Connecting with the platform, the meter and the platform can function as an ECG detector, a weight scale, and a body composition estimator. Further, the meter can alternatively connect with a chassis thus functioning as a display device of a sphygmomanometer, a stethoscope, a glucose meter, or a pulse oximeter, wherein the glucose meter and pulse oximeter have individual processor. Furthermore, the sphygmomanometer, stethoscope, glucose meter, and pulse oximeter can be selectively associated in the meter or the chassis. In case of associated in the meter, they may have no processor, but share the same processor arranged in the meter. Moreover, according to the present invention, the meter can be a cellular phone, a PDA, or the like; therefore the hospital may cooperate with the communication business and hospital to construct the real-time monitoring system for the patients.

### BRIEF DESCRIPTION OF THE DRAWINGS

Fig. 1 is a structure diagram of a multifunction health apparatus according to one embodiment of the present invention.
Fig. 2A-2F respectively show a front, top, bottom, right side, left side, and rear view of the meter of one embodiment of the present invention.
Fig. 3A-3E shows some parts of the meter according to one embodiment of the present invention.
Fig.4A-4C respectively show lead I, lead II, and lead III of the three standard leads.
Fig.5A-5C respectively show AVR Lead, AVL Lead, and AVF lead of the three augmented leads.
Fig.6 shows the standard Six Chest Leads.
Fig.7 shows that the meter connecting to the chassis is able to function as a body composition estimator, weight scale, and ECG detecting machine, according to one embodiment of the present invention.
Fig.8A-8B show that the meter connecting to the chassis is able to function as a stethoscope, sphygmomanometer, glucose meter, or pulse oximeter, according to one embodiment of the present invention; Fig. 8B is a side view of the chassis connecting with the meter.
Fig. 9 shows a wrist-type cuff or an arm-type cuff according to one embodiment of the present invention.
Figs. 10A and 10B show another embodiment of the chassis according to the present invention.
Figs. 11 and 12 show another embodiment of the meter according to the present invention.

### DESCRIPTION OF THE PREFERRED EMBODIMENT

The detailed description of the present invention will be discussed in the following embodiments, which are not intended to limit the scope of the present invention, but can be adapted for other applications. While drawings are illustrated in details, it is appreciated that the quantity of the disclosed components may be greater or less than that disclosed, except expressly restricting the amount of the components.

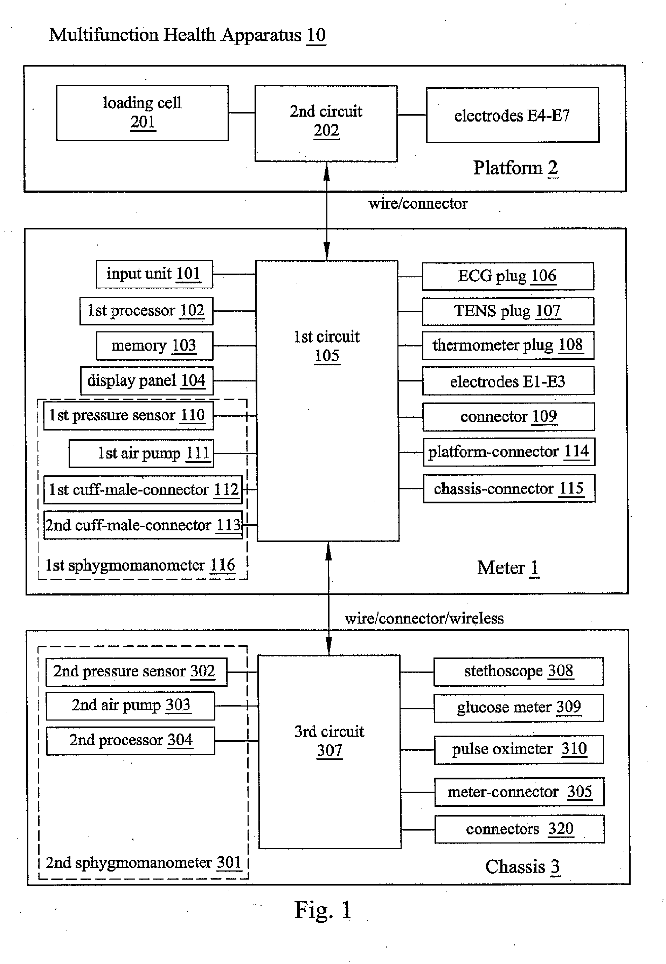

Fig.1 shows a block diagram of a multifunction health apparatus 10 of a preferred embodiment, according to the present invention. The multifunction health apparatus 10 includes three key devices-a meter 1, a platform 2, and a chassis 3. The meter 1 can optionally connect with one of the other two devices, by a well-known method such as wire, connector, or wireless communication. A display panel 104 of the meter 1 functions as a display device and control panel of the other two devices.

The meter 1 comprises an input unit 101, a first processor 102, a memory 103, the display panel 104, a first circuit 105, an ECG plug 106, a TENS plug 107, a thermometer plug 108, a connector 109, a first pressure sensor 110, a first air pump 111, a first cuff-connector 112, a second cuff-connector 113, a platform-connector 114, a chassis-connector 115, and three electrodes E1-E3. The input unit 101 is employed for inputting and outputting data; the first processor 102 for processing the data; the memory 103 for saving the data; the display panel 104 for displaying information and tested result; the first circuit 105 for transmitting, converting, and processing signal; the other components of the meter 1 will be discussed later. The platform 2 comprises a loading cell 201, a second circuit 202, and four electrodes E4-E7. The chassis 3 comprises a stethoscope 308, a glucose meter 309, a pulse oximeter 310, a third circuit 307, and a second sphygmomanometer 301. Where the second sphygmomanometer 301 comprises a second pressure sensor 302, a second air pump 303, and a second processor 304..

The multifunction health apparatus 10 is able to estimate, measure, or detect the multi-physiological parameters of human body. It also can function as a Transcutane Elektrische Neuro Stimulatie (TENS) machine for pain relief. The following paragraphs will respectively descript each function and measurement.

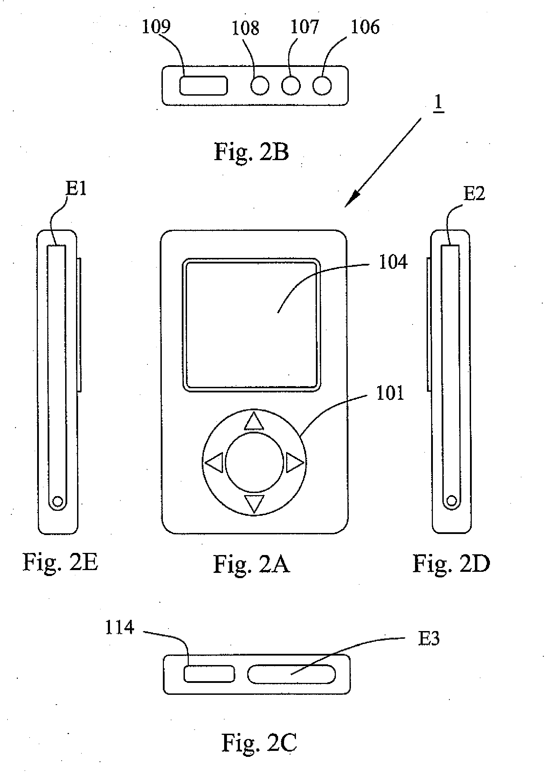

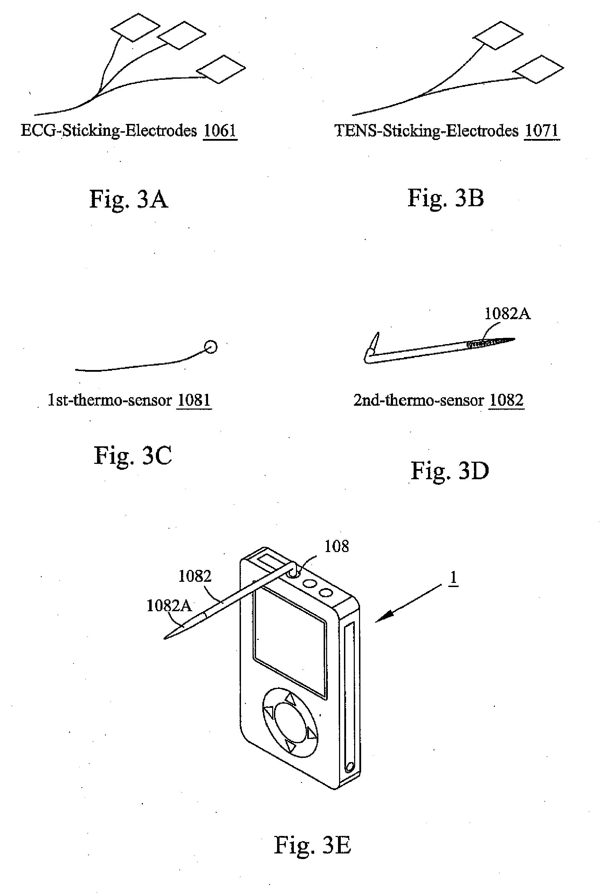

Fig.2A-Fig. 2F respectively show the front, top, bottom, right, left, and rear view of the meter 1, according to one embodiment of the present invention. Fig. 3A-Fig. 3E show some parts of the meter 1, according to Fig. 2A-Fig. 2F.

Without connecting to the platform 2 or the chassis 3, single use of the meter 1 can function as a TENS machine. The user select the TENS function and control the stimulating strength via the input unit 101. At least one pair of TENS-sticking-electrodes 1071 (Fig. 3B) is placed at appropriate pain sights on the user's body. The TENS-sticking-electrodes 1071 connect to the meter 1 via the TENS plug 107. Thus, pain relief will be started and continued after the user calls for the TENS function. If the TENS-sticking-electrodes 1071 are not applied, the electode E3 can function as the electrode for pain relief.

Without connecting to the platform 2 or the chassis 3, single use of the meter 1 can also functions as thermometer. For once measuring, the user holds a 2nd-thermo-sensor 1082 including a metal tip 1082A (Fig. 3D) into the mouth to measure the body temperature. The metal tip 1082A is responsible for detecting the body temperature. As shown in Fig. 3E, the 2nd-thermo-sensor 1082 electrically connects to the meter 1 via the thermometer plug 108 (also shown in Fig. 1). According to the embodiment, the 2nd-thermo-sensor 1082 is also a screen-touching kit of the meter 1, an alternative of entering data by touching the display panel 104 directly. If a long time monitoring is required, then the user sticks a 1st-thermo-sensor 1081 (Fig. 3C) on the body and connects the other terminal of the 1st-thermo-sensor 1081 to the meter 1 via the thermometer plug 108. Consequently the display panel 104 will show the measuring result.

According to one embodiment of the present invention, the multifunction health apparatus 10 can also functions as an ECG-detecting machine. There are four methods to detect ECG; the first employs the meter 1 only, the second employs the meter 1 with at least a set of ECG-sticking-electrodes 1061 (Fig. 3A), the third employs the meter 1 with the platform 2, and the fourth employs the platform 2 only.

Generally, the standard 12-lead electrocardiogram (ECG) represent the heart's electrical activity recorded from electrodes on the body surface. The standard 12-lead ECG includes three standard leads, three augmented leads, and six chest leads.

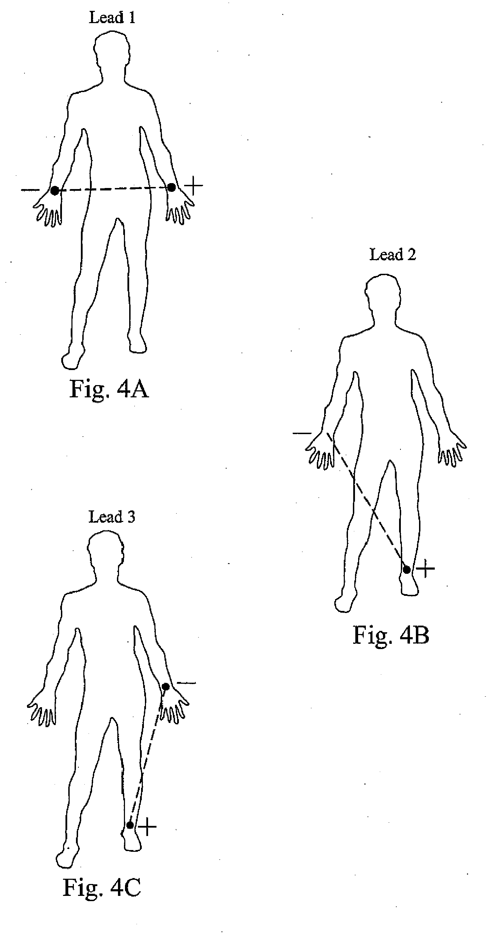

The three standard leads, also named bipolar leads, usually designated as lead I, II and III. Fig. 4A-4C shows the positions of two electrodes of each standard leads. Lead I is between the right arm and left arm electrodes, the left arm being positive; Lead II is between the right arm and left leg electrodes, the left leg being positive; Lead III is between the left arm and left leg electrodes, the left leg again being positive. Actually, when testing, the electrodes are not necessary to contact the skin in accurately position. For example, a position needing to contact at left wrist may be changed to contact at left upper arm, left lower arm, left palm, or even left leg. The ECG result will not be affected.

According to this concept, there are various variations to detect the lead I, II and III. According one embodiment of the present invention, the single use of the meter 1 can function as an ECG detector, that is, the electrodes E1, E2, and E3 can be used for detecting ECG. For example, while detecting lead I, the user holds the meter 1 by his left hand, then using the electrode E3 to contact right wrist. Thus the ECG result of lead I can be detected. In this situation, the electrodes E1 and E2 serve as positive, while the electrode E3 serves negative. The user can alternatively use right hand holds the meter 1 and use the electrode E3 to contact the left arm. In this situation, the electrodes E1 and E2 serve as negative, while the electrode E3 serve as positive. As the same concept, the meter 1 can detect the lead II and III. For example, the user holds the meter 1 by right hand and uses the electrode E3 to contact left leg, thus detecting the ECG of lead II; the user holds the meter 1 by left hand and uses electrode E3 to contact left leg, thus detecting the ECG of lead III.

The detecting methods of ECG mentioned above are suitable for short time. If longer time monitoring such as 24hour-minitoring is required, the ECG-sticking-electrodes 1061 (Fig. 3A) that are capable of sticking on the skin will be used. By sticking the electrodes of the ECG-sticking-electrodes 1061 on the positions required, connecting the other terminal to the ECG plug 106 of the meter 1 (Fig. 2B and Fig. 1), the ECG thus can be detected.

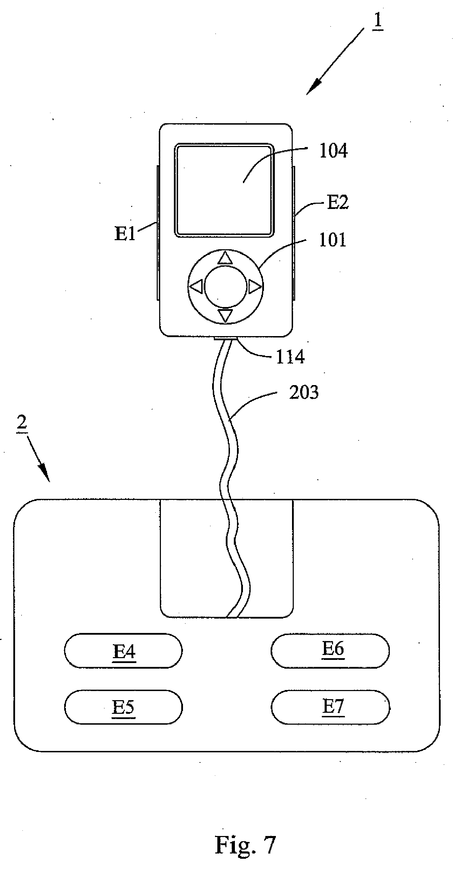

As shown in Fig. 7, connecting to the platform 2 by a wire 203 and platform-connector 114, the meter 1 can also detects the lead I, lead II, and lead III. Here one more connecter for connection between the meter 1 and the platform 2 is also applicable in another embodiment. Four electrodes-E5, E6, E7, and E8-disposed on the platform 2 will cooperate with the electrodes E1, E2, and E3 of the meter 1 to detect ECG. For testing, the user needs to step on the platform 2 and hold the meter 1 as well. For example, while detecting lead II and III, the electrodes E1 and E2 serve as negative, and E4 and E5 serve as positive. According to the design of present invention, the ECG detecting can still be proceeded, if the user merely contact one of the two same electrodes, that is, one of electrodes E4 and E5, or one of electrodes E6 and E7. Also the meter 1 employs this concept, the user need merely to contact one of the electrodes E1 and E2.

If the user steps on the platform 2, but not holds the meter 1, the single use of the platform 2 can detect the lead I and lead II, but fails to detect lead III. In this situation, the electrodes E6 and E7 serve as negative, and electrodes E4 and E5 serve as positive.

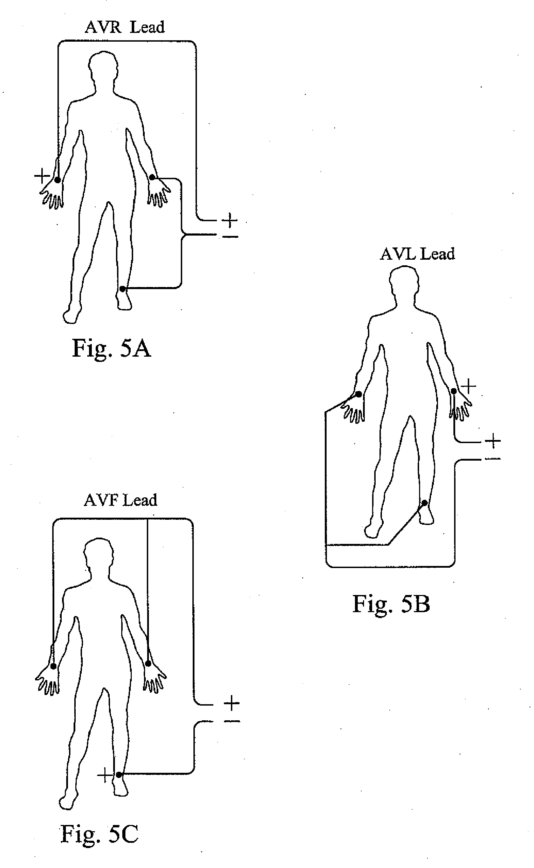

Fig.5A-Fig. 5C show the three augmented leads are AVR, AVL, and AVF lead. While detecting, both the meter 1 and the platform 2 are necessary. In one embodiment, the user uses right hand to hold the meter 1 (contact at least one of electrodes-E1 or E2, positive), uses the meter 1 to contact left wrist by the electrode E3 (negative), and steps on the platform 2 thus contacting the electrodes E4 and/or E5 by left sole (negative); therefore the AVR lead can be detected. Similarly, the AVL and AVF leads can be detected by using the same concept.

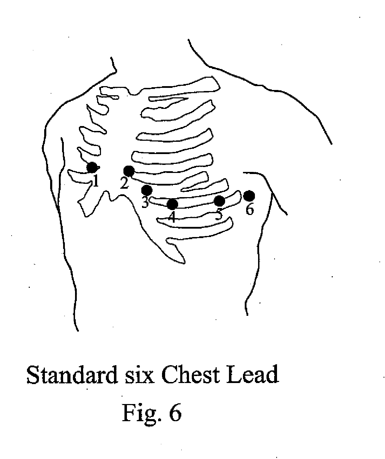

Fig. 6 shows six positions of six chest leads. The electrode E3 of the meter 1 can be used as the detecting electrode, total six-time detecting can get six ECG results of chest leads.

According bio-impedance measuring theory, it needs two pairs of electrodes to measure human whole body impedance at least. One pair of electrodes are current supply electrodes and the other one are impedance-measuring electrodes. Then we could calculate body fat condition by the bio-impedance. If we just want to measure the impedance of abdomen, it needs three pairs of electrodes. One pair of electrode contact left foot and the second of electrode contact right foot, the third contact right or left hand. Through the three pair of electrodes we could measure the body impedance from left hand to right food and left hand to left food. The difference of the two impedances should cause by the human abdomen, and then we could get the impedance of abdomen. Then we could calculate the fat condition of abdomen.

Referring to Fig. 7, the multifunction health apparatus 10 is able to estimate the body composition and weight by the meter 1 cooperating the platform 2. According to one embodiment, single frequency bioelectrical impedance analysis has been used for estimating body composition such as total body water (TBW), fat-free mass (FFM), body fat (%BF), and so forth. The embodiment uses four-electrode method to contact impedance or skin-electrode interaction.. The user stands on the platform 2 and holds the meter 1 by left hand; Simultaneously, his palm contacts the electrodes E1, his fingers contact the E2, his right sole contacts the electrodes E7, his right toes contact the E6, his left sole contacts the E5, and his left toes contact the E4. The two pairs of electrodes E2 and E6, and electrodes E2 and E4, serve as current-supply electrodes. The two pairs of electrodes E1 and E7, and electrodes E1 and E5, serve as impedance-measuring electrodes. The pair of E2 and E6, and the pair of E1 and E7, will be employed to calculate the whole body impedance, called impedance-1. The whole body impedance can calculate the body fat (%BF). The pair of E2 and E4, and the pair of E1 and E5, will be employed to calculate the body impedance, called impedance-2, which does not pass through the abdomen. The difference of the impedance-1 and the impedance-2 is caused by abdomen. It can be employed to calculate the fat condition of abdomen.

In case of the user stands on the platform 2 and holds the meter 1 by right hand; Simultaneously, his right palm contacts the electrodes E2, his fingers contact the E1, his right sole contacts the electrodes E7, his right toes contact the E6, his left sole contacts the E5, and his left toes contacts the E4. The two pairs of electrodes E1 and E6, and electrodes E1 and E4, serve as current-supply electrodes. The two pairs of electrodes E2 and E7, and electrodes E2 and E5, serve as impedance-measuring electrodes. The pair of E1 and E4, and the pair of E2 and E5, will be employed to calculate the whole body impedance, called impedance-1. The whole body impedance can calculate the body fat (%BF). The pair of E1 and E6, and the pair of E2 and E7, will be employed to calculate the body impedance, called impedance-2, which does not pass through the abdomen. The difference of the impedance-1 and the impedance-2 is caused by abdomen. It can be employed to calculate the fat condition of abdomen.

The wire 203 actually consists of several wires (not shown). Meanwhile, a loading cell 201(see Fig. 1, not shown in Fig. 7), mounted under the top face of the platform 2, measures the user's weight and transfers the measured result to the meter 1 via the wire 203 and platform-connector 114. In addition to the measured bioelectrical impedance and weight, the user must key in some parameters correlating to estimation of body composition via the input unit 101. These parameters are height, age, and sex. Certainly, this procedure can be done before or after the bioelectrical impedance and weight measurement. Therefore, the first processor 102 uses the whole body impedance, measured weight, and the inputted parameters, to estimate the user's body composition, that is, total body water (TBW), fat-free mass (FFM), body fat (%BF), and so forth. The first processor 102 transfers the estimated result to the display panel 104. According to the embodiment of the present invention, the user can estimate his body composition easily, quickly, and painlessly, and check the estimated result via the display panel 104 held by hand without bending his back.

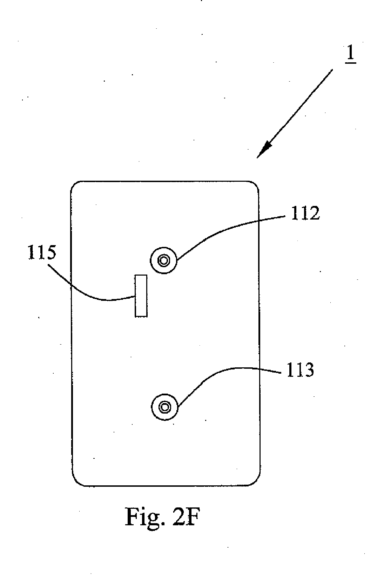

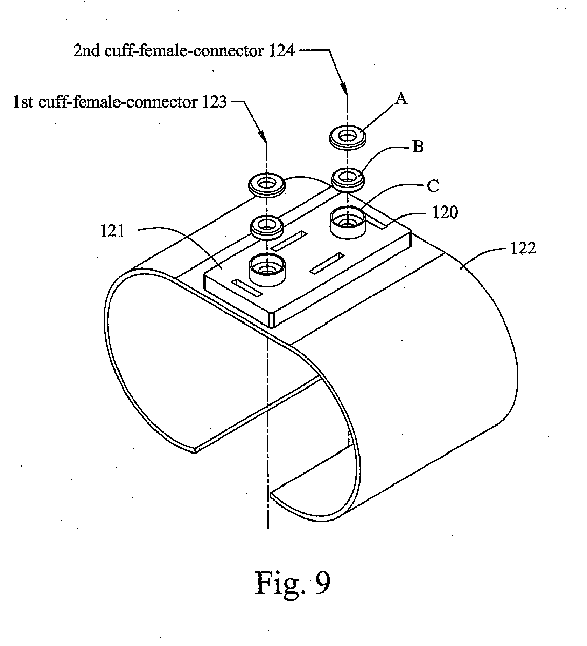

Referring to Fig. 1, Fig. 2F, and Fig. 9, the meter 1 cooperating with a cuff 122 can funtion as a 1st sphygmomanometer 116. The 1 st sphygmomanometer 116 enveloped in the dashed-line rectangle of Fig. 1 comprises a 1 st pressure sensor 110, a 1 st air pump 111, a 1st cuff-male-connector 112, a 2nd cuff-male-connector. The 1st sphygmomanometer 116 certainly comprises some common components-the input unit 101, the 1st processor 102, the memory 103, the display panel 104, and the 1st circuit 105-sharing with other functions. Referring to Fig. 9, the 1st sphygmomanometer 116 further comprises a separated cuff 122 according to one embodiment of the present invention. Where the cuff 122 shown in Fig. 9 is represented by a circler, which is made of plastic, and should be further enveloped by an air bag (not shown). The meter 1 functions as the body of 1st sphygmomanometer 116; it connects with the cuff 122 via a 1st cuff-male-connector 112 and a 2nd cuff-male-connector 113 (see Fig. 1 and Fig. 2F). The 1st cuff-male-connector 112 connects with the 1st pressure sensor 110; the 2nd cuff-male-connector 113 connects with the 1 st air pump 111; the 1st cuff-male-connector 112 plugs in a 1 st cuff-female-connector 123 of the cuff 122; the 2nd cuff-male-connector 113 plugs in a 2nd cuff-female-connector 124 of the cuff 122. In this embodiment, both of the 1st cuff-female-connector 123 and the 2nd cuff-female-connector 124 comprise a cover A, an O-ring B, and a base C. The O-ring B is placed between the cover A and base C; then the cover A and base C is welded by a suitable method, such as ultrasonic welding. Further, the base C is integrated with a pedestal 121 having four slots 120 that hooked by four corresponding hooks (not shown) extruded from the cuff 122 (circler). In other embodiments, the base C may associate with the cuff by other methods. The 1st and 2nd cuff-female-connector 123/124 can provide airtight, durable connecting with the 1st and 2nd cuff-male-connector 112/113.

It is well known that electronic sphygmomanometer can be divided into wrist type and arm type. The difference between these two types is that the later needs larger cuff, and thus larger pumping capacity of air pump. For example, the former needs a cuff inflated with 150 ml air, while the later needs 350 ml. The arm type measures the blood pressures more precise, but the wrist type is more convenient in winter, when the user wears heavy coat. Thus the user may needs to buy both types. The separated cuff 122 of the embodiment will be a great benefit to provide a solution to solve this problem. According to the embodiment of the present invention, the arm type and the wrist type sphygmomanometer can share the meter 1. There are two cuffs 122, one for wrist type, and the other for arm type. The cuff 122 using for wrist type and arm type has same structure shown in Fig. 1, but the arm type has larger circler and air bag. To distinguish them, the former is called wrist-type cuff 122, and the later is called arm-type cuff 122. The user chooses the arm type or the wrist type via the display panel 104 and input unit 101, holds the meter 1 to connect with the cuff 122 on the user's wrist or upper arm, instructs the meter 1 to measure, and finally gets the result shown in display panel 105, after the 1st processor 102 estimates the blood pressure by the corresponding program stored in the memory 103.

If longer monitoring of blood pressure is needed, user will feel uncomfortable when the meter 1 is mounted on the arm or wrist for a period of time. The present invention provides another solution to solve this problem. Referring back to Fig. 1, it shows the multifunction health apparatus 10 can also function as a 2nd sphygmomanometer 301, a stethoscope 308, a glucose meter 309, or a pulse oximeter 310 by connecting the meter 1 to the chassis 3, where the 2nd sphygmomanometer 301 comprises a second pressure sensor 302, a 2nd air pump 303, and a 2nd second processor 304.

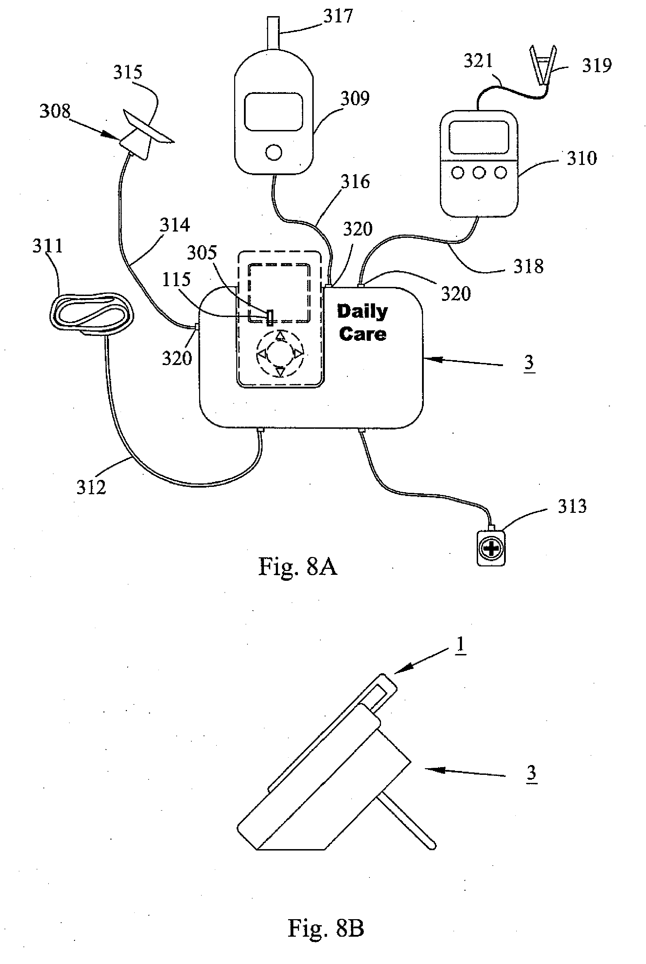

Fig. 8A shows one embodiment of the chassis 3 according to Fig. 1, the meter 1 connects to the chassis 3to function as the display device. Fig. 8B is the side view of the chassis 3. Some component arranged inside the chassis 3 are not shown such as the second pressure sensor 302, the second air pump 303, the second processor 304, and the third circuit 307.

In this embodiment, the chassis 3 connects to the meter 1 via a chassis-connector 115 and a meter-connector 305, and both of them are golden-finger connector. In other embodiments, their connecting may use any other kind of connection such as wire or wireless communication. When functioning as the second sphygmomanometer 301, the user wears the cuff 311 on his upper arm and pushes the start button 313, causing the second air pump 303 (not shown) to supply air through the air inlet valve (not shown) to the cuff 311 via the tube 312. It is noted that the cuff 311 differs from the wrist-type or arm-type cuff 122 mentioned above; the cuff 311 has a hook-and-loop fastener on its surface. When the cuff pressure reached the predetermined value, the air within the cuff 311 is turned to discharge at a constant speed via the air leakage valve (not shown). The cuff pressure decreases gradually. The second pressure sensor 302 senses the variation of the cuff pressure, transferring and converting it to the second processor 304 through the third circuit 307. According to the pressure variation of the cuff 311, the second processor 304 calculates the systolic pressure and diastolic pressure. The second processor 304 transfers the calculated result to the meter 1 through the third circuit 307 for showing in its display panel 104.

As mentioned above, the cuff 311 and arm-type cuff 122 have larger air bag, consequently larger volume that needs larger air pump to pump it. Thus it is reasonable to choose the first air pump 111 and the second air pump 303 having pumping capacity that can meet the requirement of the cuff 311 and arm-type cuff 122. For cost saving, however, the present invention provides another embodiment can further reduce the cost.

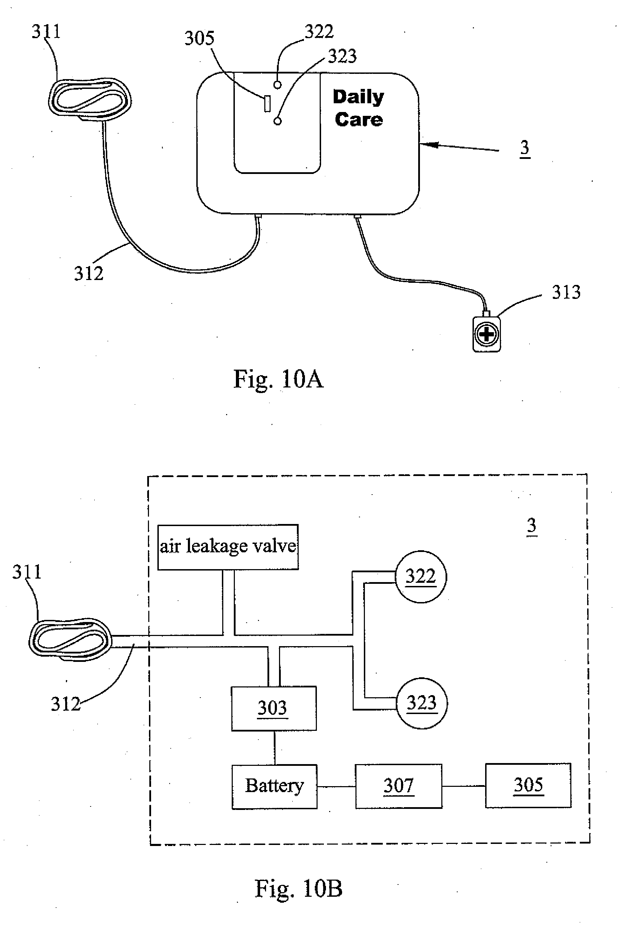

Fig. 10A and 10B show another embodiment of the present invention. Differing from the previous embodiment, the chassis 3 further comprises a first chassis-female-connector 322 and a second chassis-female-connector 323, and both of they have the same structure as the first cuff-female-connector 123 and the second cuff-female-connector 124. When the first cuff-male-connector 112 plugs in the first chassis-female-connector 322, and the second cuff-male-connector 113 plugs in the second chassis-female-connector 323, the first chassis-female-connector 322 will connect to the first pressure sensor 110 of the meter 1, and the second chassis-female-connector 323 will connect to the first air pump 111. Therefore, the second air pump 303 of the chassis 3 can be omitted. Further, the size of the meter 1 may be smallish for customer request. Thus the first air pump 111 inside the meter 1 may have pumping capacity capable of pumping the wrist-type cuff 122, but fail to pumping the arm-type cuff 122 or cuff 311, because the volume of the meter 1 may be too small to put the larger first air pump 111 inside. According to the present invention, the meter can be small as L74mm*W64mm*T24mm. Moreover, in this embodiment, for saving cost, the second air pump 303 also has pumping capacity capable of inflating the wrist-type cuff 122, but fail to pumping the arm-type cuff 122 or cuff 311. And the first air pump 111 of the meter 1 and the second air pump 303 of the chassis 3 will cooperate to inflate the cuff 311 through the tube 312. When the meter 1 connects to the chassis 3 via the chassis-connector 115 and the meter-connector 305, the first processor 102 of the meter will automatically use the program, stored in the memory 103, suitable for the arm-type, to compute the blood pressure. Alternatively, the user manually instructs the meter 1 to into the arm-type state. The meter 1 instructs the first air pump 111 to inflate the cuff 311 through the second chassis-female-connector 323, and instructs the second air pump 303 to inflate the cuff 311 through meter-connector 305, third circuit 307, and battery. When cuff pressure of the cuff 311 reaches the predetermined value, the air within the cuff 311 is turned to discharge at a constant speed via the air leakage valve. The cuff pressure decreases gradually. The

first pressure sensor 112 senses the variation of the cuff pressure, transferring and converting it to the first processor 102 through the first circuit 105. According to the pressure variation of the cuff 311, the first processor 102 calculates the systolic pressure and diastolic pressure. Finally the first processor 102 transfers the calculated result to the display panel 104.

If the second air pump 303 in the embodiment shown in Fig. 10A and 10B has pumping capacity capable of inflating the cuff 311, then the second chassis-female-connector 323 should be omitted. It is practicable in another embodiment that omits the second chassis-female-connector 323, the second processor 304, second processor, but remains the first chassis-female-connector 322.

Referring back to Fig. 8A, this embodiment shows the chassis 3 connecting to the meter 1 can also functions as a stethoscope 308. A transducer 315 is used for transferring the signal from the surface of the skin to the chassis 3 via a wire 314 and one of a plurality of the connectors 320. The transducer 315 may be any other kinds, such as a microphone or an accelerometer. After receiving the signal, a plurality of amplifiers and A/D converter of the third circuit 307 dealt with the signal, then transfer to the meter 1 to show the amplified signal in the display panel 104.

For detecting the glucose concentration of blood, the user has to connect the glucose meter 309 to the chassis 3 via a wire 316 and one of a plurality of the connectors 320. In this embodiment, the user also has to get the blood sample and puts it in the sample carrier 317. Some components inside the glucose meter 309 are not shown, such as a light source, a receiver, a biosenser, a third processor, and other necessary circuit. The light source emits a testing light to the blood sample in the sample carrier 317. The biosensor detects the optical spectrum by the reflection or transmission of the testing light. The receiver receives the optical spectrum, transferring to the third processor (not shown) to calculate the glucose concentration.
Finally the display panel 104 of the meter 1 shows the result.

According to the present invention, glucose concentration may be detected by other methods, such as the electrical chemistry method. In this case, adding some enzymes in the sample carrier 317 is necessary to test the bio-current in the blood sample.

Another embodiment of the present invention omits the third processor (not shown), instead of it; the first processor 102 inside in the meter 1 will be responsible for controlling the other components and calculating the glucose concentration, according to the signal transferred from the receiver. In this case, the wire 316 should be connected to the meter 1, not the chassis 3.

For detecting the oxygen saturation, the user uses the probe 319 to cramp his finger. The probe 319 connects the pulse oximeter 310 via a wire 321. A pair of LEDs (light emitting diodes, not shown) and at least one corresponding detector (not shown) is mounted on the probe 319. The two LEDs respectively emit the red and infrared light at predetermined wavelength to the finger cramped by the probe 319. The at least one detector detects the spectrum of light transmitted or reflected from the finger. In this embodiment, a fourth processor (not shown) is arranged inside the pulse oximeter 310 to receive the spectrum of light and thus compute the oxygen saturation and pulse beat. The computed results are transferred to the meter 1 via the wire 318 and connector 320. The display panel 104 of the meter 1 shows the result for the customer.

Again, another embodiment of the present invention omits the fourth processor (not shown). Instead of it, the first processor 102 inside in the meter 1 will be responsible for controlling the other components inside in the pulse oximeter 310, and computing the oxygen saturation and pulse beat. In this case, the wire 318 should be connected to the meter 1, not the chassis 3.

Among these embodiments mentioned above, the stethoscope 308, glucose meter 309, and pulse oximeter 310 connect to the chassis 3 or the meter 1 via the plurality of connectors 320. In case of connecting to the meter 1, only one processor is needed, that is, the first processor 102 is responsible for all control and computation works. Further, the plurality of connectors 320 is USB (Universal Serial Bus) connector, and it may be any other kinds in other embodiments. In addition, the meter 1 has at least one connector 109 for connecting to other devices such as a computer or printer. The connector 109 is also a USB connector; it may be other kinds of connectors. More, the meter 1 may be a cellular phone, a PDA (Personal Digital Assistant), MP3 (Moving Picture Experts Group audio layer-3) player, or the like; therefore the hospital may cooperate with the communication business to construct the real-time monitoring system for the patients.

In the embodiment of the present invention, the input unit 101 is button-type components; in other embodiments, the input unit 101 may be other types, such as voice-activated type, scan type, hand-written type, code data readers, or the like.

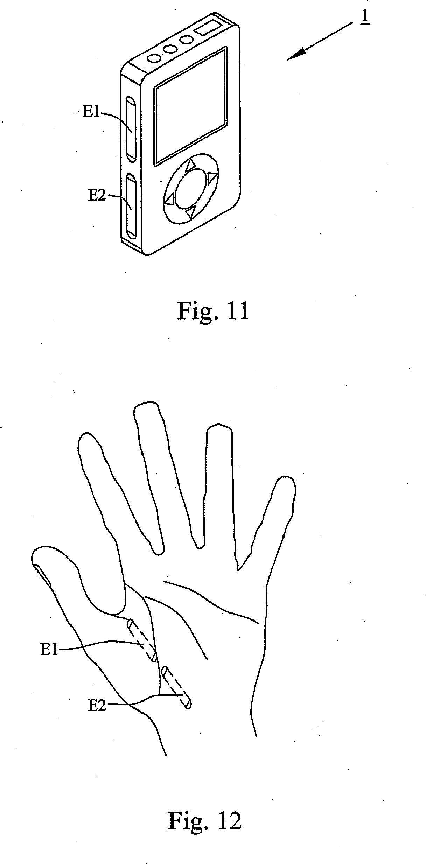

The present invention might have other modifications. For example, Figs. 11-12 show another embodiment of the meter according to the present invention. The difference between this embodiment and other embodiments is that the electrodes E1 and E2 are not arranged separately at two sides, but are arranged at one side of the meter 1, and the electrode E1 and electrode E2 remain a distance, as shown in Fig. 11. The experiments show better stability when the electrodes E1 and E2 are arranged as Fig. 11, especially for estimating the body composition. This embodiment use the similar theory as other embodiments discussed before to detect ECG and estimate body composition; the detail is omitted. Fig. 12 shows that the left palm will contact E1 and E2. In this embodiment, the electrodes E1 and E2 are arranged suitable for hold by left hand; in another embodiment, the electrodes E1 and E2 are arranged suitable for hold by right hand. Moreover, one embodiment is practicable that except the electrodes E1 and E2 are arranged at one side of the meter 1, another pair of electrodes E8 and E9 (not shown) is arranged at the other side of the meter1, and the first processor 102 decides which pair be chosen to do the work when a test is requested. As shown in Fig. 12, because both of the electrodes E1 and E2 contact the hand in palm rather than fingers, a more stable test result is reasonably expected.

According to the present invention, user can choose the functions associated in this device depending on his requirement. For example, a multifunction health apparatus 10 of one embodiment comprises the meter 1, and the meter 1 comprises a first sphygmomanometer 116, but comprises no electrodes E1-E3, because maybe the user suffers from hypertension, but not suffers from overweight, or he just considers the cost.

Although specific embodiments have been illustrated and described, it will be appreciated by those skilled in the art that various modifications may be made without departing from the scope of the present invention, which is intended to be limited solely by the appended claims.

## Claims

1. A multifunction health device, comprising:
a meter, the single use of said meter being able to optionally function as a electrocardiogram (ECG) detector, transcutaneous electrical nerve stimulation (TENS) machine, and a thermometer, said meter comprising a input unit for inputting and outputting data, a first processor for processing the data, a memory for saving the data,
a display panel for displaying information and tested result, a first circuit for transmitting, converting, and processing signal, a first electrode and a second electrode arranged separately at the two sides of said meter or both arranged at one side of said meter, a third electrode arranged at the bottom of said meter, said first electrode, second electrode, and third electrode assisting in ECG detecting and TENS.

2. The multifunction health device as recited in claim 1, further comprising:
a platform, allowing for the user stepping on, connecting with said meter being able to function as an ECG detector, a weight scale, and a body composition estimator, said platform comprising a fourth electrode for contacting the user's left-front sole, a fifth electrode for contacting the user's left-rear sole, a sixth electrode for contacting the user's right-front sole, a seventh electrode for contacting the user's right-rear sole, a loading cell arranged under the top face of said platform for sensing the user's weight, a second circuit transmitting, converting, and processing signal, then the signal being transferred to said first processor of said meter, then being displayed on said display panel of said meter.

3. The multifunction health device as recited in claim 1, further comprising:
a chassis, connecting with said meter being able to function as a sphygmomanometer, said chassis comprising a second air pump for raising pressure of a cuff worn by the user, an air inlet valve for sucking air into the air pump, an air outlet valve for air leakage of the cuff, a second pressure sensor for measuring the pressure of cuff, a second processor for processing the tested pressure signal, a third circuit for transmitting, converting, and processing signal, then the signal being transferred to said meter and being displayed on said display panel..

4. The multifunction health device as recited in claim 1, further comprising:
a first pressure sensor arranged inside said meter;
a first air pump arranged inside said meter;
a first cuff-male-connector connecting to said first pressure sensor; and
a second cuff-male-connector connecting to said first air pump.

5. The multifunction health device as recited in claim 4, further comprising:
an arm-type or wrist-type cuff separated from said meter having a first cuff-female-connector and a second cuff-female-connector, wherein both of said first cuff fernale-connector and second cuff-female-connector comprise a base associated with said cuff, a cover arranged on said base and welding with said base, and an O-ring arranged between said cover and said base;
whereby said first cuff-male-connector plugs in said first cuff-female-connector, said second cuff-male-connector plugs in said second cuff-female-connector, said first air pump inflating said arm-type or wrist-type cuff through said second cuff-male-connector and said second cuff-female-connector, said first pressure sensor sensing pressure through first cuff-male-connector and said first cuff-female-connector, said first processor estimates the result, and said display panel shows the result.

6. The multifunction health device as recited in claim 4, further comprising:
a chassis, connecting with said meter being able to function as a sphygmomanometer, said chassis comprising a second air pump for partly raising pressure of a cuff worn by the user, an air leakage valve for air leakage of the cuff, a first chassis-female-connector for connecting with said first pressure sensor via said first cuff-male-connector, a second chassis-female-connector for connecting said first air pump via second cuff-male-connector, thus said cuff being inflated via said first air pump and said second air pump, said first pressure sensor sensing the variation of cuff pressure, and a third circuit for transmitting, converting, and processing signal, then the signal being transferred to said meter and being displayed on said display panel.

7. The multifunction health device as recited in claim 1, further comprising:
a chassis, connecting with said meter being able to function as a sphygmomanometer, said chassis comprising a second air pump for raising pressure of a cuff worn by the user, an air leakage valve for air leakage of the cuff, a first chassis-female-connector for connecting with said first pressure sensor via said first cuff-male-connector, thus said first pressure sensor sensing the variation of cuff pressure, and a third circuit for transmitting, converting, and processing signal, then the signal being transferred to said meter and being displayed on said display panel.

8. The multifunction health device as recited in any of the claims 3, 6 or 7, wherein the chassis further comprises at least one of the connectors for connecting a stethoscope, a glucose meter, or a pulse oximeter, said glucose meter and pulse oximeter having individual processor, said stethoscope, glucose meter, and pulse oximeter using said meter as display device.

9. The multifunction health device as recited in claim 1, if said meter is single use to detect ECG, the first electrode, second electrode, and third electrode are employed to detect the three standard lead (lead I, lead II, lead III) and six chest lead.

10. The multifunction health device as recited in claim 1, wherein said meter further comprises
a set of ECG-sticking-electrodes as an auxiliary part for ECG detecting including three standard lead (lead I, lead II, lead III), AVR lead, AVL lead, AVF lead, and six chest lead, and/or
a first-thermo-sensor as an auxiliary part for temperature measuring, when functioning as a thermometer, said meter connects with said first-thermo-sensor that sticks on the local area of the user, and/or
a second-thermo-sensor as an auxiliary part for alternative of temperature measuring, when functioning as a thermometer, said meter connects with said second-thermo-sensor having a metal tip held in the user's mouth to detect the user's body temperature, and/or
at least one pair of TENS-sticking-electrodes as an auxiliary part for TENS.

11. The multifunction health device as recited in claim 10, wherein said second-thermo-sensor is also a screen-touching kit of said meter.

12. The multifunction health device as recited in claim 1, when functioning as a TENS machine, said meter uses said third electrode to contact the local area of user for pain relief.

13. The multifunction health device as recited in claim 1, wherein the user's body composition at least including body water (TBW), fat-free mass (FFM), and body fat (%BF) are estimated, after the user inputs some personal information, at least including height, age, and sex, via said input unit.

14. The multifunction health device as recited in claim 1, wherein said meter further receive a measured signal from a stethoscope, a glucose meter, or a pulse oximeter, said first processor processing the measured signal, said first circuit transmitting, converting, and processing the signal, and said display panel displaying the result for the user.

15. The multifunction health device as recited in claim 1, wherein said meter further receive a measured signal from a stethoscope, a glucose meter, or a pulse oximeter, said stethoscope, a glucose meter, or a pulse oximeter having individual processor for processing the signal, said first circuit transmitting, converting, and processing the signal, and said display panel displaying the result for the user.

16. The multifunction health device as recited in claim 1, if said first electrode and a second electrode are both arranged at one side of said meter, then said meter further comprises an eighth electrode and a ninth electrode arranged at the other side of said meter.

17. The multifunction health device as recited in claim 1, wherein said meter comprises a cellular phone, a PDA (Personal Digital Assistant), or a MP3 (Moving Picture Experts Group audio layer-3) player.
